# EUROPÄISCHE PATENTANMELDUNG

(11) **EP 4 104 896 A1**
(43) Veröffentlichungstag der Anmeldung: **21.12.2022**
(21) Anmeldenummer: 21180065.1
(22) Anmeldetag: 17.06.2021
(51) Int. Cl.: A61N 1/375

(54) **IMPLANTIERBARES MEDIZINISCHES GERÄT MIT EINEM STEUERGERÄT UND DARAN ANGEORDNETEN STECKVERBINDERANSCHLÜSSEN**

(71) Anmelder: BIOTRONIK SE & Co. KG, 12359 Berlin (DE)
(72) Erfinder: Kolberg, Gernot, 12161 Berlin (DE); Dörr, Thomas, 12437 Berlin (DE)
(74) Vertreter: Biotronik Corporate Services SE

(57) **Zusammenfassung**

Ein implantierbares medizinisches Gerät (1) umfasst ein Steuergerät (10), das ein Gehäuse (100) und eine Mehrzahl von Steckverbinderanschlüssen (102-104) aufweist, wobei das Gehäuse (100) durch eine umfänglich um eine Mittelpunktsachse (M) erstreckte Umfangsfläche (107) begrenzt ist, und eine Mehrzahl von an die Steckverbinderanschlüsse (102-104) anschließbaren Elektrodenleitungen (11, 12, 13), die jeweils einen Steckverbinder (112, 122, 132) zum steckenden Verbinden mit einem zugeordneten der Steckverbinderanschlüsse (102-104) aufweisen. Dabei ist vorgesehen, dass ein erster der Steckverbinderanschlüsse (102-104) und ein zweiter der Steckverbinderanschlüsse (102-104) zum Anstecken einer ersten der Elektrodenleitungen (11, 12, 13) und einer zweiten der Elektrodenleitungen (11, 12, 13) unterschiedlich ausgerichtet und/oder an unterschiedlichen, um die Mittelpunktachse (M) zueinander versetzten Orten an der Umfangsfläche (107) angeordnet sind.

## Beschreibung

Die vorliegende Erfindung betrifft ein implantierbares medizinisches Gerät nach dem Oberbegriff des Anspruchs 1 sowie ein Verfahren zum Herrichten eines implantierbaren medizinischen Geräts.

Ein derartiges implantierbares medizinisches Gerät umfasst ein Steuergerät, das ein Gehäuse und eine Mehrzahl von Steckverbinderanschlüssen aufweist, wobei das Gehäuse durch eine umfänglich um eine Mittelpunktachse erstreckte Umfangsfläche begrenzt ist. An die Steckverbinderanschlüsse ist eine Mehrzahl von Elektrodenleitungen anschließbar, die jeweils einen Steckverbinder zum steckenden Verbinden mit einem zugeordneten der Steckverbinderanschlüsse aufweisen.

Ein solches implantierbares medizinisches Gerät kann beispielsweise als kardiales Stimulationssystem, zum Beispiel als CRT-System zur sogenannten kardialen Resynchronisationstherapie (Englisch "Cardiac Resynchronisation Therapy", kurz CRT) ausgebildet sein. Bei einigen Patienten mit einer chronischen Herzschwäche (so genannter Herzinsuffizienz) ist die natürliche Erregung zur Kontraktion der Herzkammern gestört. Bei einem sogenannten Linksschenkelblock kann hierbei insbesondere die Erregung der linken Herzkammer (linker Ventrikel) derart beeinträchtigt sein, dass die linke Herzkammer nur unzureichend und zudem nicht synchron mit der rechten Herzkammer kontrahiert. Eine Folge ist, dass die Herzkammern nicht mehr in einer aufeinander abgestimmten Weise arbeiten, das Herz somit unregelmäßig schlägt und dadurch die Pumpleistung des Herzens reduziert ist. Ziel der kardialen Resynchronisationstherapie ist es somit, die Erregung der Herzkammern so zu beeinflussen, dass die Synchronität zwischen den Herzkammern wiederhergestellt wird, indem die rechte und linke Herzkammer durch eingespeiste elektrische Erregung zeitgleich stimuliert werden.

Ein implantierbares medizinisches Gerät verwendet, zum Beispiel bei Ausgestaltung als kardiales Stimulationssystem, beispielsweise mehrere Elektrodenleitungen, die gemeinsam mit dem Steuergerät implantiert und von denen zum Beispiel eine in die rechte Herzkammer (rechter Ventrikel) und eine andere in die linke Herzkammer (linker Ventrikel) verlegt werden. Eine dritte Elektrodenleitung kann zusätzlich beispielsweise im rechten Vorhof (rechtes Atrium) angeordnet sein, um eine Abstimmung mit einer Vorhofaktivität zu ermöglichen. Weitere Elektrodenleitungen können verwendet werden und an anderen Orten implantiert werden.

Ein solches implantierbares medizinisches Gerät kann beispielsweise mit einer reinen Herzschrittmacherfunktion ausgestaltet sein (zum Beispiel in Form eines sogenannten CRT-P-Systems). Weil Patienten mit einer chronischen Herzschwäche häufig auch einem erhöhten Risiko eines plötzlichen Herzstillstands (plötzlicher Herztod) unterliegen, kann ein implantierbares medizinisches Gerät zusätzlich aber auch eine Defibrillator-Funktion zur hochenergetischen Anregung zum Zwecke der Defibrillation aufweisen (zum Beispiel in Form eines sogenannten CRT-D-Systems).

Ein solches implantierbares medizinisches Gerät kann in anderer Ausgestaltung auch als Überwachungsgerät zum Aufzeichnen und Überwachen bestimmter Parameter ausgebildet sein und hierzu zum Beispiel über Elektrodenleitungen kardiale Signale detektieren.

Bei der Implantation eines solchen implantierbaren medizinischen Geräts werden die an das Steuergerät angeschlossenen Elektrodenleitungen gemeinsam mit dem Steuergerät implantiert und dabei so im Körper verlegt, dass die Elektrodenleitungen sich hin zu einem bestimmungsgemäßen Ort, zum Beispiel im Herzen des Patienten, erstrecken. Es besteht hierbei ein Bedürfnis danach, das Verlegen der Elektrodenleitungen vom Steuergerät weg zu erleichtern, insbesondere auch vor dem Hintergrund, dass bei der Explantation Elektrodenleitungen aus dem Körper zu entnehmen sind, was erschwert sein kann, wenn die Elektrodenleitungen in komplizierter Weise, zum Beispiel unter Schlaufenbildung, von dem Steuergerät weg verlegt und im Körper eingewachsen sind.

Aufgabe der vorliegenden Erfindung ist es, ein implantierbares medizinisches Gerät und ein Verfahren zum Herrichten eines implantierbaren medizinischen Geräts zur Verfügung zu stellen, die ein einfaches, platzsparendes Verlegen von Elektrodenleitungen vom Steuergerät weg ermöglichen.

Diese Aufgabe wird durch einen Gegenstand mit den Merkmalen des Anspruchs 1 gelöst.

Demnach sind ein erster der Steckverbinderanschlüsse und ein zweiter der Steckverbinderanschlüsse zum Anstecken einer ersten der Elektrodenleitungen und einer zweiten der Elektrodenleitungen unterschiedlich ausgerichtet und/oder an unterschiedlichen, um die Mittelpunktachse zueinander versetzten Orten an der Umfangsfläche angeordnet.

Dadurch, dass die Steckverbinderanschlüsse an dem Gehäuse des Steuergeräts unterschiedlich ausgerichtet und/oder um die Mittelpunktachse zueinander versetzt sind, wird ermöglicht, die Elektrodenleitungen in unterschiedlicher Ausrichtung und/oder an unterschiedlichen Orten an die Steckverbinderanschlüsse des Steuergeräts anzuschließen. Durch unterschiedliche Ausrichtung der Steckverbinderanschlüsse wird insbesondere einfach möglich, Elektrodenleitungen so an das Steuergerät anzuschließen, dass die Elektrodenleitungen entlang unterschiedlicher Richtungen von dem Steuergerät weg verlegt werden können. Weil Elektrodenleitungen somit in einfacher Weise in unterschiedlicher Ausrichtung und/oder Platzierung von dem zum Beispiel im Brustkorb zu implantierenden Steuergerät abgehen können, können die Elektrodenleitungen hin zu einem Zielgebiet, zum Beispiel durch Einführung in ein Gefäßsystem, verlegt werden, insbesondere ohne dass Elektrodenleitungen in Schlaufen oder engen Wendungen zu legen sind.

Die Elektrodenleitungen sind über ihre Steckverbinder an die Steckverbinderanschlüsse des Steuergeräts anzuschließen. Die Steckverbinderanschlüsse des Steuergeräts sind hierbei zum Beispiel vorbestimmten Elektrodenleitungen, beispielsweise einer Elektrodenleitung zur linksventrikulären Stimulation, einer Elektrodenleitung zur rechtsventrikulären Stimulation und einer Elektrodenleitung zur Stimulation im rechten Atrium, zugeordnet. Über eine Steuereinrichtung des Steuergeräts werden die Steckverbinderanschlüsse und darüber die angeschlossenen Elektrodenleitungen angesteuert, um zum Beispiel elektrische Stimulationspulse in bestimmungsgemäßer Weise abzugeben. Steckverbinderanschlüsse des Steuergeräts, die bestimmungsgemäß hin zu einem bestimmten Zielgebiet zu verlegenden Elektrodenleitungen zugeordnet sind, können hierbei so an dem Steuergerät platziert und/oder ausgerichtet sein, dass ein Verlegen der Elektrodenleitungen von dem Steuergerät weg in einfacher und platzsparender Weise möglich ist.

In einer Ausgestaltung sind die Steckverbinderanschlüsse des Steuergeräts nicht fest jeweils einer bestimmten Elektrodenleitung, zum Beispiel einer Elektrodenleitung zur rechtsventrikulären Stimulation oder einer Elektrodenleitung zur Stimulation im rechten Atrium, zugeordnet. Die Steckverbinderanschlüsse können vielmehr zum Beispiel so konfigurierbar sein, dass an jeden Steckverbinderanschluss unterschiedliche Elektrodenleitungen angeschlossen werden können, wobei durch Programmierung des Steuergeräts festgelegt wird, welche Funktion eine an einen bestimmten Steckverbinderanschluss angeschlossene Elektrodenleitung erfüllen soll, zum Beispiel für eine rechtsventrikuläre Stimulation oder eine Stimulation im rechten Atrium. Entsprechend der Programmierung werden zum Beispiel Stimulationspulse an einen Steckverbinderanschluss und darüber an die an den Steckverbinderanschluss angeschlossene Elektrodenleitung abgegeben, sodass das Steuergerät variabel konfigurierbar und variabel mit Elektrodenleitungen bestückbar ist.

In einer Ausgestaltung sind der erste der Steckverbinderanschlüsse und der zweite der Steckverbinderanschlüsse derart an dem Gehäuse des Steuergeräts angeordnet, dass die erste der Elektrodenleitungen entlang einer ersten Steckrichtung mit dem ersten der Steckverbinderanschlüsse und die zweite der Elektrodenleitungen entlang einer von der ersten Steckrichtung unterschiedlichen, zweiten Steckrichtung mit dem zweiten der Steckverbinderanschlüsse verbindbar ist. Die Steckverbinderanschlüsse sind somit unterschiedlich ausgerichtet, sodass die Elektrodenleitungen mit ihren Steckverbindern entlang unterschiedlicher Steckrichtungen an die Steckverbinderanschlüsse angeschlossen werden können.

Die Steckrichtungen können hierbei beispielsweise um einen Winkel zwischen 30° und 180° (gemessen um eine zur Mittelpunktachse parallele Achse oder eine zur Mittelpunktachse senkrechte oder gegebenenfalls auch schräge Achse) zueinander winkelbeabstandet sein, sodass die Elektrodenleitungen in unterschiedlichen Winkeln an das Steuergerät anzuschließen sind.

Sind die Steckrichtungen um 180° zueinander winkelbeabstandet, so sind die Steckrichtungen entgegengesetzt zueinander gerichtet, sodass die Elektrodenleitungen in entgegengesetzten Steckrichtungen an das Steuergerät anzuschließen sind.

In einer Ausgestaltung sind die erste der Elektrodenleitungen und die zweite der Elektrodenleitungen entlang unterschiedlich gerichteter Abgangsachsen von dem Gehäuse erstreckt, wenn die erste der Elektrodenleitungen und die zweite der Elektrodenleitungen an den ersten der Steckverbinderanschlüsse und den zweiten der Steckverbinderanschlüsse angeschlossen sind. Die Elektrodenleitungen sind üblicherweise als längserstreckte flexible Leitungen ausgebildet, wobei an einem proximalen Ende einer jeden Elektrodenleitung ein Steckverbinder zum steckenden Verbinden mit einem zugeordneten der Steckverbinderanschlüsse des Steuergeräts und an einem anderen, distalen Ende eine Elektrodenanordnung zum Abgeben und/oder Empfangen von elektrischen Signalen angeordnet ist. Die Elektrodenleitungen sind üblicherweise biegsam, wobei eine einer Elektrodenleitung jeweils zugeordnete Abgangsachse durch eine solche Achse definiert ist, entlang derer die Elektrodenleitung im Bereich des in einen jeweils zugeordneten Steckverbinderanschluss des Steuergeräts eingesteckten Steckverbinders von dem Steuergerät abgeht. Die Kabelabgangsachse entspricht insofern beispielsweise der Steckrichtung, entlang derer die Elektrodenleitung mit ihrem Steckverbinder in den zugeordneten Steckverbinderanschluss einzustecken ist.

In einer Ausgestaltung sind der erste der Steckverbinderanschlüsse und der zweite der Steckverbinderanschlüsse entlang der Mittelpunktachse und/oder radial zur Mittelpunktachse zueinander versetzt. Die Steckverbinderanschlüsse können somit beispielsweise, betrachtet entlang der Mittelpunktachse, nebeneinander angeordnet sein. Zusätzlich oder alternativ können die Steckverbinderanschlüsse auch radial versetzt zueinander angeordnet sein und somit unterschiedliche radiale Abstände zur Mittelpunktachse aufweisen. Zusätzlich sind die Steckverbinderanschlüsse unterschiedlich ausgerichtet und/oder an unterschiedlichen Orten am Steuergerät platziert und ermöglichen somit ein Anstecken von Elektrodenleitungen entlang unterschiedlicher Richtungen und/oder an unterschiedlichen Orten am Steuergerät.

In einer Ausgestaltung sind der erste der Steckverbinderanschlüsse und der zweite der Steckverbinderanschlüsse um einen Winkel zwischen 30° und 180° um die Mittelpunktachse zueinander versetzt. Die Orte, an denen die Steckverbinderanschlüsse im Bereich der Umfangsfläche am Gehäuse des Steuergeräts angeordnet sind, weisen somit einen Winkelabstand zueinander auf, gemessen um die Mittelpunktachse.

Sind die Steckverbinderanschlüsse an der Umfangsfläche so zueinander beabstandet, dass sie einen Winkelabstand von 180° zueinander aufweisen, so sind die Steckverbinderanschlüsse an diametral zueinander angeordneten, voneinander abgewandten Seiten des Gehäuses angeordnet, sodass an unterschiedlichen Seiten des Gehäuses je eine oder mehrere Elektrodenleitungen angeschlossen werden können.

In einer Ausgestaltung weist das Gehäuse einen Anschlussblock auf, an dem der erste der Steckverbinderanschlüsse und der zweite der Steckverbinderanschlüsse gemeinsam angeordnet sind. In diesem Fall sind die Steckverbinderanschlüsse somit an einem gemeinsamen Ort im Bereich der Umfangsfläche an dem Gehäuse des Steuergeräts angeordnet, wobei die Steckverbinderanschlüsse in diesem Fall unterschiedlich zueinander ausgerichtet sind und somit Elektrodenleitungen entlang unterschiedlicher Steckrichtungen an die Steckverbinderanschlüsse angeschlossen werden können.

An dem Anschlussblock können (genau) zwei Steckverbinderanschlüsse angeordnet sein, die unterschiedlich zueinander ausgerichtet sind. Denkbar und möglich ist aber auch, dass einem Anschlussblock mehr als zwei Steckverbinderanschlüsse zum Anschließen von mehr als zwei Elektrodenleitungen angeordnet sind, wobei zumindest zwei der Steckverbinderanschlüsse unterschiedlich zueinander ausgerichtet sind.

Der Anschlussblock kann beispielsweise durch eine Ausformung an dem Gehäuse ausgebildet sein, die radial gegenüber anderen Abschnitten des Gehäuses vorsteht. Während das Gehäuse beispielsweise eine kreisscheibenartige Grundform aufweisen kann (betrachtet im Querschnitt quer zur Mittelpunktachse), kann der Anschlussblock radial zu dieser kreisscheibenartigen Grundform vorstehen. Ein Steckverbinderanschluss des Anschlussblocks kann dabei beispielsweise ein Anschließen einer Elektrodenleitung entlang einer tangentialen Steckrichtung (bezogen auf die kreisscheibenartige Grundform) ermöglichen.

In anderer Ausgestaltung kann das Gehäuse mehrere Anschlussblöcke aufweisen, die um die Mittelpunktachse zueinander versetzt sind und an denen der erste der Steckverbinderanschlüsse und der zweite der Steckverbinderanschlüsse angeordnet sind. Ein jeder Anschlussblock kann hierbei einen oder mehrere Steckverbinderanschlüsse aufweisen. Dadurch, dass die Anschlussblöcke an unterschiedlichen Orten an der Umfangsfläche des Gehäuses angeordnet sind, können Elektrodenleitungen unterschiedlich am Gehäuse platziert und unterschiedlich zum Gehäuse verlegt werden.

Die Anschlussblöcke können wiederum beispielsweise durch je eine Ausformung an dem Gehäuse ausgebildet sein, die radial gegenüber anderen Abschnitten des Gehäuses vorsteht. Das Gehäuse kann wiederum beispielsweise eine kreisscheibenartige Grundform aufweisen (betrachtet im Querschnitt quer zur Mittelpunktachse), wobei die Anschlussblöcke radial zu dieser kreisscheibenartigen Grundform vorstehen.

Die Mittelpunktachse weist beispielsweise durch einen Volumenschwerpunkt des Gehäuses des Steuergeräts. Beispielsweise kann das Gehäuse eine flache Grundform aufweisen, indem das Gehäuse durch senkrecht zur Mittelpunktachse ausgerichtete, zueinander im Wesentlichen parallele Seitenflächen begrenzt ist. Die Mittelpunktachse weist somit senkrecht zur flachen Grundform des Gehäuses. Umfänglich um die Mittelpunktachse ist das Gehäuse durch die Umfangsfläche begrenzt, wobei die Steckverbinderanschlüsse im Bereich der Umfangsfläche am Gehäuse angeordnet sein können und dabei zum Beispiel in ihrer Ausrichtung oder in ihrer Platzierung an der Umfangsfläche zueinander unterschiedlich sind.

In einer Ausgestaltung bildet das Steuergerät einen Pulsgenerator für ein kardiales Stimulationssystem aus. Während das Steuergerät in Form des Pulsgenerators beispielsweise im Bereich des Brustkorbs, zum Beispiel subkutan, zu implantieren ist, können die Elektrodenleitungen vom Steuergerät weg hin zu einem Zielgebiet, zum Beispiel im Herzen des Patienten, verlegt werden. Über den Pulsgenerator können Stimulationspulse erzeugt und über die Elektrodenleitungen in das Zielgebiet abgegeben werden, sodass eine Stimulationsfunktion insbesondere im Herzen des Patienten ausgeübt werden kann.

Das implantierbare medizinische Gerät kann in diesem Zusammenhang beispielsweise als kardiales Stimulationssystem, zum Beispiel als CRT-System zur sogenannten kardialen Resynchronisationstherapie (Englisch "Cardiac Resynchronisation Therapy", kurz CRT) ausgebildet sein.

Die Steckverbinderanschlüsse des Steuergeräts können zum Beispiel genormt sein, und ebenso sind die an den Elektrodenleitungen angeordneten Steckverbinder zum steckenden Verbinden mit den Steckverbinderanschlüssen genormt.

Beispielsweise können die Steckverbinderanschlüsse jeweils vierpolig mit vier elektrischen Kontaktelementen ausgestaltet sein. Entsprechend sind die Steckverbinder der Elektrodenleitungen ausgestaltet, sodass ein jeder Steckverbinder an einen zugeordneten vierpoligen Steckverbinderanschluss des Steuergeräts angeschlossen werden kann.

Beispielsweise kann es sich bei den Steckverbinderanschlüssen jeweils um einen sogenannten IS4-Anschluss, der nach der Norm ISO 27186:2010 (erste Ausgabe aus dem März 2010) genormt ist, oder einen DF4-Anschluss handeln.

Denkbar und möglich ist auch, dass einer oder mehrere der Steckverbinderanschlüsse als einpolige oder zweipolige Steckverbinderanschlüsse mit jeweils einem elektrischen Kontaktelement oder zwei Kontaktelementen ausgebildet sind, beispielsweise in Form einpoliger oder zweipoliger IS-1-Anschlüsse (nach ISO 27186:2010).

Die Aufgabe wird auch gelöst durch ein Verfahren zum Herrichten eines implantierbaren medizinischen Geräts, aufweisend: Bereitstellen eines Steuergerät, das ein Gehäuse und eine Mehrzahl von Steckverbinderanschlüssen aufweist, wobei das Gehäuse durch eine umfänglich um eine Mittelpunktsachse erstreckte Umfangsfläche begrenzt ist; und steckendes Verbinden von Steckverbindern einer Mehrzahl von Elektrodenleitungen mit den Steckverbinderanschlüssen. Dabei ist vorgesehen, dass eine erste der Elektrodenleitungen und eine zweite der Elektrodenleitungen an einen ersten der Steckverbinderanschlüsse und einen zweiten der Steckverbinderanschlüsse, die unterschiedlich ausgerichtet und/oder an unterschiedlichen, um die Mittelpunktachse zueinander versetzten Orten an der Umfangsfläche angeordnet sind, angesteckt werden.

Die vorangehend für das implantierbare medizinische Gerät beschriebenen Vorteile und vorteilhaften Ausgestaltungen finden analog auch auf das Verfahren Anwendung, sodass diesbezüglich auf die vorangehenden Ausführungen verwiesen werden soll.

Der der Erfindung zugrunde liegende Gedanke soll nachfolgend anhand der in den Figuren dargestellten Ausführungsbeispiele näher erläutert werden. Es zeigen:
- Fig. 1: eine Ansicht des menschlichen Herzens mit einem implantierten medizinischen Gerät zum Beispiel in Form eines CRT-Systems;
- Fig. 2: eine schematische Ansicht eines Anschlussblocks eines Steuergeräts eines implantierbaren medizinischen Geräts;
- Fig. 3: eine Ansicht eines Ausführungsbeispiels eines Steuergeräts eines implantierbaren medizinischen Geräts mit daran angeschlossenen Elektrodenleitungen;
- Fig. 4: eine Ansicht eines weiteren Ausführungsbeispiels eines Steuergeräts;
- Fig. 5: eine Ansicht eines wiederum anderen Ausführungsbeispiels eines Steuergeräts;
- Fig. 6: eine Ansicht eines wiederum anderen Ausführungsbeispiels eines Steuergeräts; und
- Fig. 7: eine schematische Draufsicht auf das Steuergerät, zum Beispiel nach der Ausgestaltung gemäß Fig. 3.

Im Folgenden werden Ausführungsbeispiele mit Bezug auf die Figuren beschrieben. In den Figuren sind hierbei Bauteile gleicher Funktion mit gleichen Bezugszeichen versehen.

Bereits an dieser Stelle sei angemerkt, dass die beschriebenen Ausführungsbeispiele nicht als beschränkend für die vorliegende Erfindung zu verstehen sind, sondern lediglich der Illustration dienen.

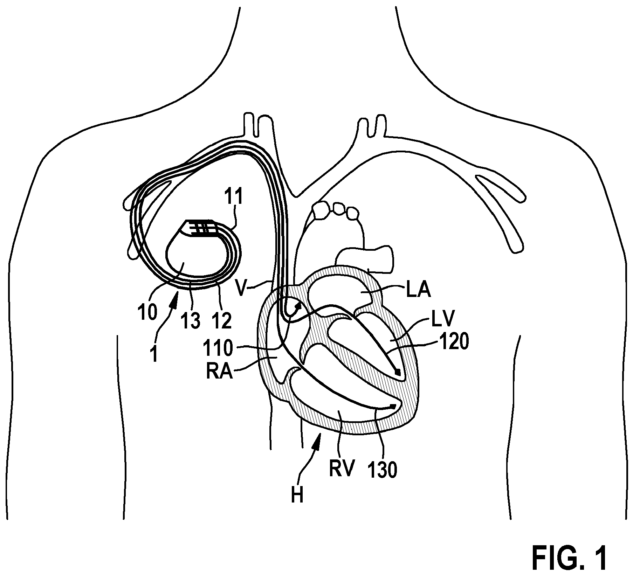

Fig. 1 zeigt in einer schematischen Ansicht das Herz H eines Patienten mit einem implantierten medizinischen Gerät 1 zum Beispiel in Form eines kardialen Schrittmachersystems. Das medizinische Gerät 1 weist ein Steuergerät 10 auf, an das Elektrodenleitungen 11, 12, 13 angeschlossen sind. Das Steuergerät 10 ist zusammen mit den Elektrodenleitungen 11, 12, 13 im Patienten implantiert derart, dass sich die Elektrodenleitungen 11, 12, 13 ausgehend vom Steuergerät 10 durch eine obere Hohlvene V hin zum Herzen H erstrecken und zu unterschiedlichen Stimulationsorten im Herzen H verlegt sind.

Bei dem dargestellten Beispiel sind drei Elektrodenleitungen 11, 12, 13 an das Steuergerät 10 angeschlossen. Das Steuergerät 10 ist beispielsweise subkutan im Bereich des Schlüsselbeins des Patienten implantiert. Von dem Steuergerät 10 sind die Elektrodenleitungen 11, 12, 13 derart verlegt, dass die Elektrodenleitungen 11, 12, 13 mit ihren distalen Enden 110, 120, 130 zum Beispiel im rechten Atrium RA (Elektrodenleitung 11 mit dem distalen Ende 110), im linken Ventrikel LV (Elektrodenleitung 12 mit dem distalen Ende 120) und im rechten Ventrikel RV (Elektrodenleitung 13 mit dem distalen Ende 130) zu liegen kommen und somit über durch das Steuergerät 10 generierte und in die Elektrodenleitungen 11, 12, 13 eingeleitete Stimulationspulse eine Stimulation an unterschiedlichen Stimulationsorten im Herzen H erfolgen kann.

Im Rahmen zum Beispiel einer kardialen Resynchronisationstherapie erfolgt eine Stimulation im linken Ventrikel LV - wobei der linke Ventrikel LV z. B. über das Herzvenensystem vom rechten Atrium aus erreichbar ist - und im rechten Ventrikel RV, sodass durch die Stimulation eine Synchronität der Ventrikelaktivität bewirkt werden kann. Auf diese Weise soll bei Patienten mit einer chronischen Herzschwäche (Herzinsuffizienz) die Pumpleistung des Herzens H erhöht werden.

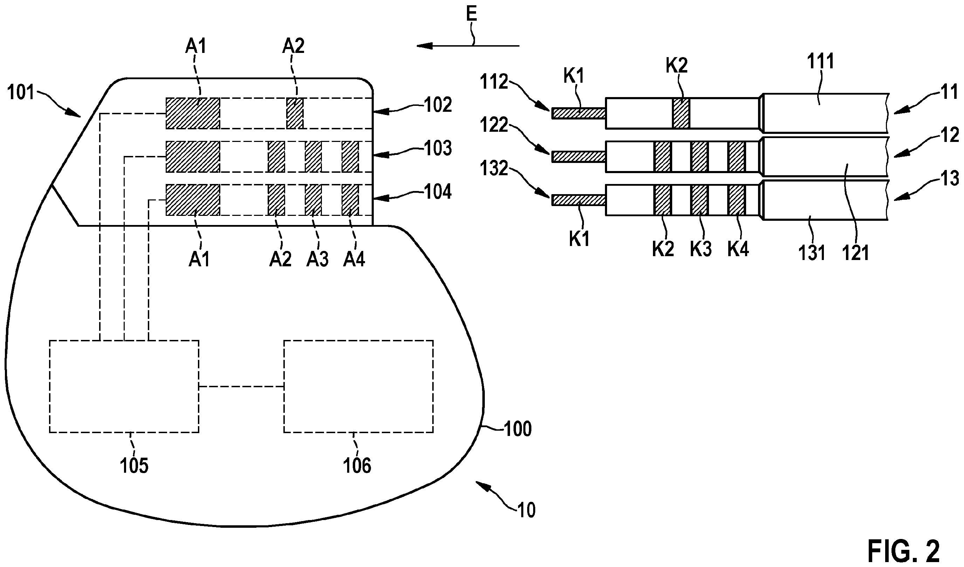

Wie schematisch in Fig. 2 dargestellt ist, weist das Steuergerät 10 ein Gehäuse 100 auf, in dem elektrische und elektronische Komponenten in Form einer Steuereinrichtung 105 und einer Energieversorgungseinrichtung 106 in Form einer Batterie eingefasst und gekapselt sind.

An dem Gehäuse 100 ist ein Anschlussblock 101 angeordnet, der Steckverbinderanschlüsse 102, 103, 104 in Form von Buchsenverbindern aufweist, in die die Elektrodenleitungen 11, 12, 13 mit zugeordneten Steckverbindern 112, 122, 132 in Form von Steckern eingesteckt werden können, um eine elektrische Kontaktierung zwischen den Elektrodenleitungen 11, 12, 13 und den Steckverbinderanschlüssen 102, 103, 104 und somit mit dem Steuergerät 10 zu erreichen.

Die Steckverbinderanschlüsse 102, 103, 104 sind vorzugsweise genormt und sind beispielsweise als IS-1- oder IS4-Anschlüsse nach ISO 27186:2010 ausgestaltet. Die Steckverbinderanschlüsse 102, 103, 104 weisen jeweils elektrische Kontaktelemente A1-A4 auf, wobei ein erster Steckverbinderanschluss 102 beispielsweise zwei elektrische Kontaktelemente A1, A2 und die anderen beiden Steckverbinderanschlüsse 103, 104 jeweils vier elektrische Kontaktelemente A1-A4 aufweisen können. Der Steckverbinderanschluss 102 ist somit als zweipoliger Anschluss ausgestaltet, während die anderen Steckverbinderanschlüsse 103, 104 als vierpolige Anschlüsse ausgebildet sind.

Entsprechend der Ausgestaltung der Steckverbinderanschlüsse 102, 103, 104 sind die Steckverbinder 112, 122, 132 der Elektrodenleitungen 11, 12, 13 beispielsweise als IS-1-Stecker (Steckverbinder 112) oder IS4-Stecker (Steckverbinder 122, 132) ausgestaltet und weisen zwei elektrische Kontaktelemente K1, K2 (zweipoliger Steckverbinder 112) oder vier elektrische Kontaktelemente K1-K4 (vierpolige Steckverbinder 122, 132) auf.

Die Kontaktelemente A1-A4 auf Seiten der Steckverbinderanschlüsse 102, 103, 104 und die Kontaktelemente K1-K4 auf Seiten der Steckverbinder 112, 122, 132 sind derart ausgestaltet, dass beim steckenden Verbinden eine paarweise elektrische Kontaktierung zwischen den jeweils zugeordneten Kontaktelementen A1-A4, K1-K4 hergestellt wird.

Im Rahmen eines herkömmlichen implantierbaren medizinischen Geräts 1, wie es in Fig. 2 beispielhaft dargestellt ist, sind die Steckverbinderanschlüsse 102, 103, 104 an einem gemeinsamen Anschlussblock 101 angeordnet und ermöglichen ein Anstecken von Elektrodenleitungen 11, 12, 13 entlang einer gemeinsamen, gleichgerichteten Steckrichtung E. Die Steckverbinderanschlüsse 102, 103, 104 sind dabei jeweils einer bestimmten Elektrodenleitung 11, 12, 13 zugeordnet. So dient ein Steckverbinderanschluss 102 zum Anschließen einer Elektrodenleitung 11, die in das rechte Atrium RA zu verlegen ist. Ein zweiter Steckverbinderanschluss 103 dient demgegenüber zum Anschließen einer linksventrikulären Elektrodenleitung 12. Ein Steckverbinderanschluss 104 dient zum Anschließen einer rechtsventrikulären Elektrodenleitung 13.

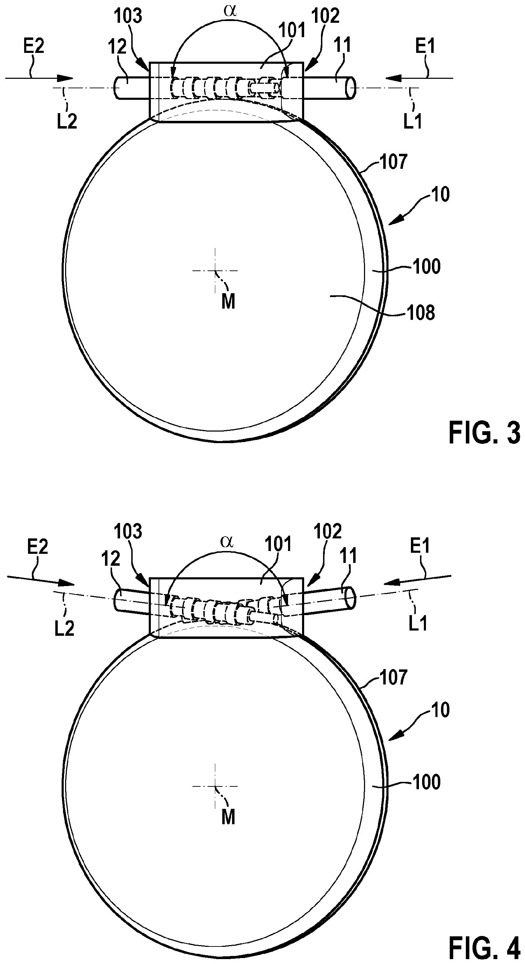

Bei einem in Fig. 3 dargestellten Ausführungsbeispiel eines Steuergeräts 10 sind demgegenüber Steckverbinderanschlüsse 102, 103 an einem gemeinsamen Anschlussblock 101 angeordnet, der von einem (im Querschnitt quer zu einer Mittelpunktachse M) kreisscheibenförmigen Gehäuse 100 radial vorsteht. Die Steckverbinderanschlüsse 102, 103 sind hierbei derart entgegengesetzt zueinander ausgerichtet, dass sie einen Winkel α von 180° zueinander beschreiben und zugeordnete Elektrodenleitungen 11, 12 entlang entgegengesetzter Steckrichtungen E1, E2 an die Steckverbinderanschlüsse 102, 103 des Anschlussblocks 101 angesteckt werden können, um die Elektrodenleitungen 11, 12 mit dem Steuergerät 10 zu verbinden.

Die Steckrichtungen E1, E2 sind bei dem dargestellten Ausführungsbeispiel entgegengesetzt zueinander gerichtet. Die Steckrichtungen E1, E2 sind hierbei jeweils tangential zu der kreisscheibenartigen Grundform des Gehäuses 100 gerichtet.

Weil die Steckverbinderanschlüsse 102, 103 unterschiedlich ausgerichtet sind, indem sie einen Winkel α von 180° zueinander beschreiben, können die Elektrodenleitungen 11, 12 entlang unterschiedlicher Kabelabgangsachsen L1, L2 von dem Steuergerät 10 weg verlegt werden. Dies ermöglicht, die Elektrodenleitungen 11, 12 so an dem Steuergerät 10 anzuordnen, dass die Elektrodenleitungen 11, 12 ausgehend von dem Implantationsort des Steuergeräts 10 in einfacher Weise hin zu einem Zielgebiet verlegt werden können.

Die Steckverbinderanschlüsse 102, 103 können hierbei jeweils einer bestimmten Elektrodenleitung 11, 12, zum Beispiel einer rechtsventrikulären Elektrodenleitung oder einer rechts atrialen Elektrodenleitung, zugeordnet sein, sodass eine definierte Elektrodenleitung an einen bestimmten Steckverbinderanschluss 102, 103 anzuschließen ist. Alternativ kann das Steuergerät 10 aber auch konfigurierbar sein, sodass den Steckverbinderanschlüssen 102, 103 unterschiedliche Funktionen zugewiesen werden können und somit an einen Steckverbinderanschluss 102, 103 unterschiedliche Elektrodenleitungen 11, 12 angeschlossen werden können, wobei die Konfiguration des Steuergeräts 10 für den Betrieb durch Programmierung bestimmt werden kann.

Dies ermöglicht beispielsweise, eine rechtsventrikuläre Elektrodenleitung entweder an den Steckverbinderanschluss 102 oder den Steckverbinderanschluss 103 anzuschließen, wobei abhängig davon, welche Elektrodenleitung 11, 12 an welchen Steckverbinderanschluss 102, 103 angeschlossen wird, das Steuergerät 10 entsprechend programmiert wird, um im Betrieb Steuerimpulse für eine bestimmte Funktion, zum Beispiel eine rechtsventrikuläre Stimulation, an einen entsprechenden Steckverbinderanschluss 102, 103, an den die entsprechende Elektrodenleitung 11, 12 angeschlossen ist, abzugeben.

In einem anderen Ausführungsbeispiel, dargestellt in Fig. 4, sind an einem Anschlussblock 101 zwei Steckverbinderanschlüsse 102, 103 angeordnet, die um einen Winkel α kleiner 180° zueinander winkelbeabstandet sind. Der Winkel α kann beispielsweise zwischen 30° und 180° liegen. Die Steckverbinderanschlüsse 102, 103 sind somit schräg zueinander ausgerichtet. Entsprechend sind Steckrichtungen E1, E2, entlang derer die Elektrodenleitungen 11, 12 an die Steckverbinderanschlüsse 102, 103 anzuschließen sind, schräg zueinander gerichtet.

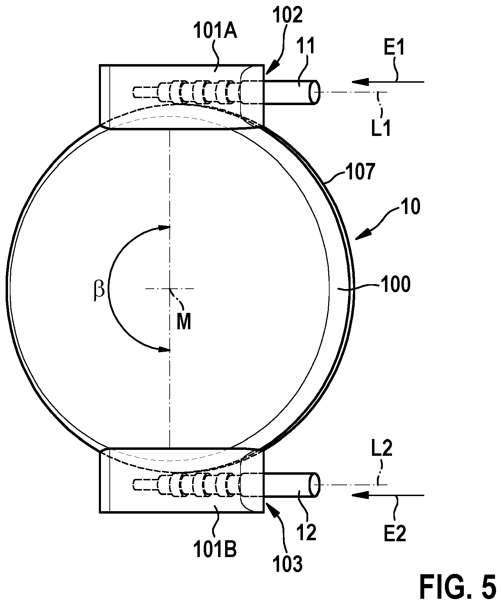

Während bei dem Ausführungsbeispiel gemäß Fig. 3 und 4 ein Anschlussblock 101 vorgesehen ist, der zwei Steckverbinderanschlüsse 102, 103 aufweist, sind bei dem Ausführungsbeispiel gemäß Fig. 5 zwei unterschiedliche Anschlussblöcke 101A, 101B vorgesehen, die jeweils radial von dem kreisscheibenförmigen Gehäuse 100 des Steuergeräts 10 vorstehen und jeweils (zumindest) einen Steckverbinderanschluss 102, 103 aufweisen. Die Anschlussblöcke 101A, 101B sind hierbei an unterschiedlichen, diametral zur Mittelpunktachse M zueinander angeordneten Seiten des Gehäuses 100 angeordnet und entsprechend um einen Winkel β von 180° um die Mittelpunktachse M zueinander winkelversetzt an einer Umfangsfläche 107 des Gehäuses 100 angeordnet.

Bei dem Ausführungsbeispiel gemäß Fig. 5 sind die Steckrichtungen E1, E2, in die Elektrodenleitungen 11, 12 an die Steckverbinderanschlüsse 102, 103 anzuschließen sind, gleichgerichtet, wobei durch die Anordnung der Steckverbinderanschlüsse 102, 103 an unterschiedlichen Orten an der Umfangsfläche 107 des Gehäuses 101 die Elektrodenleitungen 11, 12 entfernt voneinander am Gehäuse 100 anzuschließen sind.

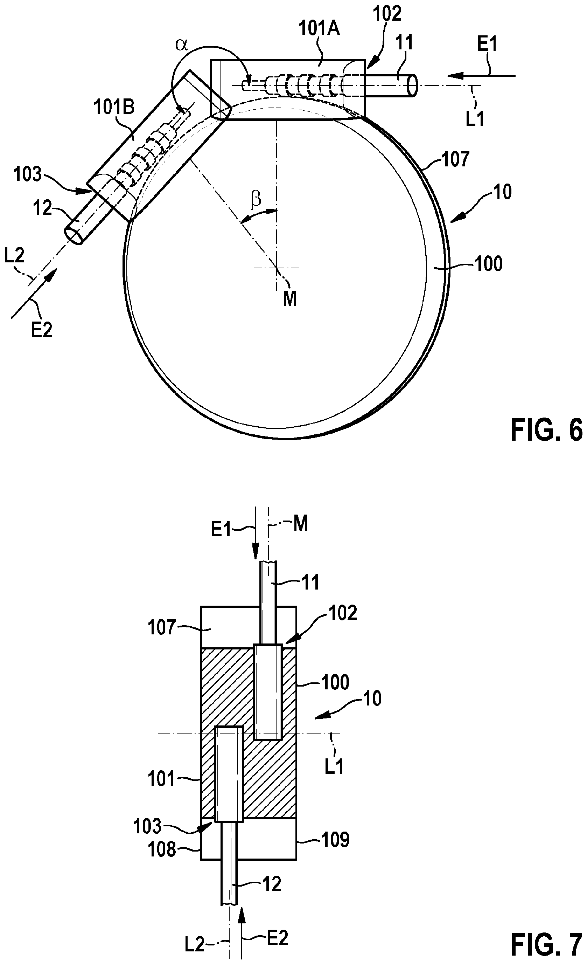

Während bei dem Ausführungsbeispiel gemäß Fig. 5 die Anschlussblöcke 101A, 101B diametral gegenüberliegend zueinander angeordnet sind, sind bei dem Ausführungsbeispiel gemäß Fig. 6 Anschlussblöcke 101A, 101B um einen Winkel β kleiner als 180° zueinander winkelversetzt an der Umfangsfläche 107 des Gehäuses 100 angeordnet. Der Winkel β kann zum Beispiel zwischen 30° und 180° betragen.

Bei dem Ausführungsbeispiel gemäß Fig. 5 und genauso bei dem Ausführungsbeispiel gemäß Fig. 6 erfolgt ein Anstecken der Elektrodenleitungen 11, 12 tangential zu der kreisscheibenförmigen Grundform des Gehäuses 100.

Bei sämtlichen Ausführungsbeispielen weist das Gehäuse 100 eine flache Grundform auf, die im Querschnitt zum Beispiel - wie dargestellt - kreisscheibenförmig ausgebildet ist. Wie dies schematisch in Fig. 7 dargestellt ist, kann das Gehäuse 100 hierbei durch senkrecht zur Mittelpunktachse M erstreckte Seitenflächen 108, 109 begrenzt sein, wobei die axiale Dicke des Gehäuses 100, gemessen entlang der Mittelpunktachse M, klein ist im Vergleich zum Durchmesser des Gehäuses 100 senkrecht zur Mittelpunktachse M.

Die Mittelpunktachse M kann beispielsweise durch einen geometrischen Volumenschwerpunkt des Gehäuses 100 weisen und ist senkrecht zu den Seitenflächen 108, 109 des Gehäuses 100 gerichtet.

Die Steckverbinderanschlüsse 102, 103 eines Anschlussblocks 101 (wie bei den Ausführungsbeispielen gemäß Fig. 3 und 4) können beispielsweise, wie dies aus Fig. 7 ersichtlich ist, betrachtet entlang der Mittelpunktachse M nebeneinander angeordnet sein. Denkbar und möglich ist aber auch, dass die Steckverbinderanschlüsse 102, 103 radial zueinander versetzt sind.

Bei den Ausführungsbeispielen gemäß Fig. 3 und 4 können an einem jeden Anschlussblock 101 (genau) zwei Steckverbinderanschlüsse 102, 103 angeordnet sein, wobei auch denkbar und möglich ist, an dem Anschlussblock 101 mehr als zwei Steckverbinderanschlüsse 102, 103 zum Anstecken von mehr als zwei Elektrodenleitungen 11, 12 vorzusehen.

Bei den Ausführungsbeispielen gemäß Fig. 5 und 6 können an jedem Anschlussblock 101A, 101B auch mehr als ein Steckverbinderanschluss 102, 103 angeordnet sein.

Die Steckverbinderanschlüsse 102, 103 des Steuergeräts 10 können genormt sein und beispielsweise als vierpolige IS4-Anschlüsse oder als einpolige oder zweipolige IS-1-Anschlüsse ausgebildet sein.

Bei Ausgestaltung des Steuergeräts 10 als Pulsgenerator in einem Stimulationssystem, zum Beispiel für eine kardiale Stimulation, werden über eine Steuereinrichtung 105 (siehe Fig. 1) elektrische Stimulationspulse generiert und über die Steckverbinderanschlüsse 102, 103 an die zugeordneten Elektrodenleitungen 11, 12 abgegeben. Bei einem vierpoligen Steckverbinderanschluss zum Beispiel für eine linksventrikuläre Elektrodenleitung oder eine rechtsventrikuläre Elektrodenleitung können hierbei in mehrpoliger Weise unterschiedliche zeitversetzte Stimulationspulse generiert und abgegeben werden, wobei eine zeitliche Verzögerung zwischen zum Beispiel den linksventrikulären Stimulationspulsen einstellbar ist.

Das implantierbare medizinische Gerät 1 kann als reines Schrittmachersystem ausgebildet sein (sogenanntes CRT-P-System). Alternativ kann das implantierbare medizinische Gerät auch eine Defibrillator-Funktion aufweisen (sogenanntes CRT-D-System). Wiederum alternativ kann das implantierbare medizinische Gerät 1 eine Überwachungs- und/oder andere Stimulationsfunktion, gegebenenfalls auch eine nicht kardiale Stimulationsfunktion, aufweisen.

Der der Erfindung zugrunde liegende Gedanke ist nicht auf die vorangehend beschriebenen Ausführungsbeispiele beschränkt, sondern lässt sich auch in anderer Weise verwirklichen.

Die Anschlüsse des Steuergeräts können auch anders als beschrieben ausgebildet sein. Beispielsweise kann das Steuergerät lediglich mehr als zwei, insbesondere auch mehr als drei Anschlüsse aufweisen.

### Liste der Bezugszeichen

- 1: Implantierbares medizinisches Gerät
- 10: Steuergerät (Generator)
- 100: Gehäuse
- 101, 101A, 101B: Anschlussblock
- 102-104: Steckverbinderanschluss
- 105: Steuereinrichtung
- 106: Energieversorgungseinrichtung
- 107: Umfangsfläche
- 108, 109: Seitenfläche
- 11: Elektrode
- 110: Distales Ende
- 111: Proximales Ende
- 112: Steckverbinder
- 12: Elektrode
- 120: Distales Ende
- 121: Proximales Ende
- 122: Steckverbinder
- 13: Elektrode
- 130: Distales Ende
- 131: Proximales Ende
- 132: Steckverbinder
- α, β: Winkel
- A1-A4: Kontaktelement
- E, E1, E2: Steckrichtung
- H: Herz
- K1-K4: Kontaktelement
- L1, L2: Abgangsachse (Elektrodenleitungenabgangsrichtung)
- LA: Linkes Atrium
- LV: Linkes Ventrikel
- M: Mittelpunktachse (geometrischer Schwerpunkt)
- RA: Rechtes Atrium
- RV: Rechtes Ventrikel
- V: Obere Hohlvene

## Patentansprüche

1. Implantierbares medizinisches Gerät (1), mit
einem Steuergerät (10), das ein Gehäuse (100) und eine Mehrzahl von Steckverbinderanschlüssen (102-104) aufweist, wobei das Gehäuse (100) durch eine umfänglich um eine Mittelpunktsachse (M) erstreckte Umfangsfläche (107) begrenzt ist, und
einer Mehrzahl von an die Steckverbinderanschlüsse (102-104) anschließbaren Elektrodenleitungen (11, 12, 13), die jeweils einen Steckverbinder (112, 122, 132) zum steckenden Verbinden mit einem zugeordneten der Steckverbinderanschlüsse (102-104) aufweisen,
**dadurch gekennzeichnet, dass** ein erster der Steckverbinderanschlüsse (102-104) und ein zweiter der Steckverbinderanschlüsse (102-104) zum Anstecken einer ersten der Elektrodenleitungen (11, 12, 13) und einer zweiten der Elektrodenleitungen (11, 12, 13) unterschiedlich ausgerichtet und/oder an unterschiedlichen, um die Mittelpunktachse (M) zueinander versetzten Orten an der Umfangsfläche (107) angeordnet sind.

2. Implantierbares medizinisches Gerät (1) nach Anspruch 1, **dadurch gekennzeichnet, dass** der erste der Steckverbinderanschlüsse (102-104) und der zweite der Steckverbinderanschlüsse (102-104) derart an dem Gehäuse (100) des Steuergeräts (10) angeordnet sind, dass die erste der Elektrodenleitungen (11, 12, 13) entlang einer ersten Steckrichtung (E1) mit dem ersten der Steckverbinderanschlüsse (102-104) und die zweite der Elektrodenleitungen (11, 12, 13) entlang einer von der ersten Steckrichtung (E1) unterschiedlichen, zweiten Steckrichtung (E2) mit dem zweiten der Steckverbinderanschlüsse (102-104) verbindbar ist.

3. Implantierbares medizinisches Gerät (1) nach Anspruch 2, **dadurch gekennzeichnet, dass** die erste Steckrichtung (E1) und die zweite Steckrichtung (E2) einen Winkel (α) zwischen 30° und 180° zueinander aufweisen.

4. Implantierbares medizinisches Gerät (1) nach Anspruch 2 oder 3, **dadurch gekennzeichnet, dass** die erste Steckrichtung (E1) und die zweite Steckrichtung (E2) entgegengesetzt zueinander gerichtet sind.

5. Implantierbares medizinisches Gerät (1) nach einem der Ansprüche 2 bis 4, **dadurch gekennzeichnet, dass** die erste der Elektrodenleitungen (11, 12, 13) und die zweite der Elektrodenleitungen (11, 12, 13) entlang unterschiedlich gerichteter Abgangsachsen (L1, L2) von dem Gehäuse (10) erstreckt sind, wenn die erste der Elektrodenleitungen (11, 12, 13) und die zweite der Elektrodenleitungen (11, 12, 13) an den ersten der Steckverbinderanschlüsse (102-104) und den zweiten der Steckverbinderanschlüsse (102-104) angeschlossen sind.

6. Implantierbares medizinisches Gerät (1) nach einem der vorangehenden Ansprüche, **dadurch gekennzeichnet, dass** der erste der Steckverbinderanschlüsse (102-104) und der zweite der Steckverbinderanschlüsse (102-104) entlang der Mittelpunktachse (M) und/oder radial zur Mittelpunktsachse (M) zueinander versetzt sind.

7. Implantierbares medizinisches Gerät (1) nach einem der vorangehenden Ansprüche, **dadurch gekennzeichnet, dass** der erste der Steckverbinderanschlüsse (102-104) und der zweite der Steckverbinderanschlüsse (102-104) um einen Winkel (β) zwischen 30° und 180° um die Mittelpunktachse (M) zueinander versetzt an der Umfangsfläche (107) angeordnet sind.

8. Implantierbares medizinisches Gerät (1) nach einem der vorangehenden Ansprüche, **dadurch gekennzeichnet, dass** der erste der Steckverbinderanschlüsse (102-104) und der zweite der Steckverbinderanschlüsse (102-104) an diametral zueinander angeordneten Seiten des Gehäuses (100) angeordnet sind.

9. Implantierbares medizinisches Gerät (1) nach einem der Ansprüche 1 bis 8, **dadurch gekennzeichnet, dass** das Gehäuse (10) einen Anschlussblock (101) aufweist, an dem der erste der Steckverbinderanschlüsse (102-104) und der zweite der Steckverbinderanschlüsse (102-104) gemeinsam angeordnet sind.

10. Implantierbares medizinisches Gerät (1) nach einem der Ansprüche 1 bis 8, **dadurch gekennzeichnet, dass** das Gehäuse (10) unterschiedliche, um die Mittelpunktachse (M) zueinander versetzte Anschlussblöcke (101A, 101B) aufweist, an denen der erste der Steckverbinderanschlüsse (102-104) und der zweite der Steckverbinderanschlüsse (102-104) angeordnet sind.

11. Implantierbares medizinisches Gerät (1) nach einem der vorangehenden Ansprüche, **dadurch gekennzeichnet, dass** das Gehäuse (100) eine flache Grundform, mit das Gehäuse (100) begrenzenden, senkrecht zur Mittelpunktachse (M) ausgerichteten, zueinander im Wesentlichen parallelen Seitenflächen (108, 109), aufweist.

12. Implantierbares medizinisches Gerät (1) nach einem der vorangehenden Ansprüche, **dadurch gekennzeichnet, dass** das Steuergerät (10) einen Pulsgenerator für ein kardiales Stimulationssystem ausbildet.

13. Implantierbares medizinisches Gerät (1) nach einem der vorangehenden Ansprüche, **dadurch gekennzeichnet, dass** der erste der Steckverbinderanschlüsse (102-104) und/oder der zweite der Steckverbinderanschlüsse (102-104) als einpoliger oder zweipoliger IS-1-Anschluss oder als vierpoliger IS4-Anschluss oder DF4-Anschluss ausgebildet sind.

14. Verfahren zum Herrichten eines implantierbaren medizinischen Geräts (1), aufweisend:
Bereitstellen eines Steuergeräts (10), das ein Gehäuse (100) und eine Mehrzahl von Steckverbinderanschlüssen (102-104) aufweist, wobei das Gehäuse (100) durch eine umfänglich um eine Mittelpunktsachse (M) erstreckte Umfangsfläche (107) begrenzt ist, und
steckendes Verbinden von Steckverbindern (112, 122, 132) einer Mehrzahl von Elektrodenleitungen (11, 12, 13) mit den Steckverbinderanschlüssen (102-104),
**dadurch gekennzeichnet, dass** eine erste der Elektrodenleitungen (11, 12, 13) und eine zweite der Elektrodenleitungen (11, 12, 13) an einen ersten der Steckverbinderanschlüsse (102-104) und einen zweiten der Steckverbinderanschlüsse (102-104), die unterschiedlich ausgerichtet und/oder an unterschiedlichen, um die Mittelpunktachse (M) zueinander versetzten Orten an der Umfangsfläche (107) angeordnet sind, angesteckt werden.
